# EUROPEAN PATENT APPLICATION

(11) **EP 4 066 876 A1**
(43) Date of publication of application: **05.10.2022**
(21) Application number: 20892403.5
(22) Date of filing: 25.11.2020
(51) Int. Cl.: A61M 25/00, A61M 25/02, A61F 2/78

(54) **RETAINING DEVICE FOR A URINARY CATHETER AND ASSEMBLY COMPRISING A RETAINING DEVICE WITH A CATHETER**

(30) Priority: 25.11.2019 ES 201931039
(71) Applicant: Rethink Medical SL, 35017 Las Palmas de Gran Canaria (ES)
(72) Inventor: LUQUE GONZÁLEZ, Manuel, 35017 LAS PALMAS DE GRAN CANARIA (ES); RUIZ CANALES, Jaime, 35017 LAS PALMAS DE GRAN CANARIA (ES)
(74) Representative: Ponti & Partners, S.L.P
(86) International application number: PCT/ES2020/070734
(87) International publication number: WO 2021/105539

(57) **Abstract**

The retention device is configured to be coupled to the end outer part of a urinary catheter or feeding catheter of the type having a "T" shaped valve, the retention device comprising a body with:
- a first portion extending in a longitudinal direction "X" and having an inner recess for receiving and accommodating an end part of a urinary catheter or feeding tube where the first portion is closed above by an upper portion comprising an anti-leakage element of the open end of the catheter or tube connecting the catheter,
- a second portion extending in a second direction "Y", transverse to the longitudinal direction "X", wherein the second portion comprises an inner recess extending along the transverse direction "Y", and wherein the second portion is open at one of its ends, while at the opposite end it comprises an anti-opening wall, and
- a fastening element, configured to releasably fasten the retention device to an element external to the user's body.

This allows the user to perform any type of sports activity with the catheter fitted and placed inside the support without any risk of accidental action.

The invention also concerns the assembly formed by this retention device and a catheter.

## Description

The present invention relates to a removable retention device, suitable for use in combination with a catheter, preferably a urinary catheter, and which is capable of retaining in a fixed position a catheter on an outside part of the user's body, which allows the user to perform any type of sports activity with the catheter and the support in place, without there being any risk of accidental action. Alternatively, to a urinary catheter, the retention device could also be used in combination with a feeding catheter, for example, those having a "T" valve, i.e., of the sliding valve type with two or more switch positions: open, closed, etc.

Secondly, the present invention also concerns the assembly formed by this retention device and a catheter.

### Background of the invention

With regard to urinary catheters or urinary probes, these are known in the state of the art with multiple configurations and typologies. Such urinary catheters are used, for example, in hospitals for users who, due to an operation or because of their age, are no longer able to leave their bed. Urinary catheters of this type are also used in other bladder emptying disorders.

Within urinary catheters there are transurethral catheters, of which there are different types; for example, there are single-use catheters, which are removed again from the urethra after a single emptying of the urinary bladder, or permanent catheters that, in general, are configured as two-way catheters in which a balloon is arranged at the tip of the urinary catheter, the urinary catheter having a channel for the diversion of urine and a channel for filling the balloon when the urinary catheter is inserted. Using the balloon, the urinary catheter is then fixed in the urethra in a durable way.

Moreover, three-way probes or catheter, called washing probes, are also known. Unlike two-way probes, such urinary probes also have an additional channel through which washing solutions can be introduced into the urinary bladder. Said three-way probes are used, for example, in the case of severe bleeding in the urinary bladder to prevent an open channel of blood in the urinary bladder by washing the urinary bladder.

European patent no. EP2872194A1, of the same holder, discloses an improved urinary catheter (10), which presents a flexible tube (12), the flexible tube (12) having an introduction section (14) for introducing it through a urethra into a urinary bladder, and the flexible tube (12) having, at the opposite end, a connection section (70a-70b) for connecting connection elements, wherein through the introduction section (14) fluid can be evacuated from the urethra in the connection section (18), a valve (71-72) that can be closed being arranged between the introduction section (14) and the connection section (70a-70b), such that the fluid outlet from the connection section (70a-70b) can be prevented when the valve is closed (71-72), characterized in that the valve (71-72) is configured as a slide valve and has three switching positions, the valve (71-72) being closed in a closed position, and in that the valve (71-72) is air-permeable in a central position, and in that the valve (71-72) is open in a release position. When the valve is in the open position, it allows patients to use a conventional toilet or connect the device to a urine bag temporarily or permanently, according to their needs; when the valve is in the closed position, it prevents the loss of the bladder reflex and gives patients the option of not using any urine collection bag and being able to carry the catheter with an active and healthy lifestyle; and when the valve is in the control position, it allows nurses to control the urine flow during the insertion, ensuring that once the urine reaches the valve at the end of the catheter tube, it cannot leave the catheter unless the nurses decide to do so, selecting the "open position".

The use of these urinary catheters has the drawback that it significantly limits the physical activity of the user when wearing the urinary catheter, since with the urinary catheter placed the part external to the user's body is free, that is to say, without being attached to a fixed part. In addition, the urinary catheter (10) disclosed in European patent No. EP2872194A1 also does not have any accessory or support to protect it from possible blows that may damage it, nor to prevent the traction of the tube of the catheter that generally causes injuries and bleeding to the user.

It is therefore necessary to provide a new design not included in the prior art that solves the drawbacks found therein, providing a retention device, in particular to protect a urinary catheter or a feeding catheter, for example, a urinary catheter such as that described in European patent no. EP2872194A1, and at the same time to allow a user to comfortably perform physical activity (such as a sports activity), for example, while standing, with the catheter in the closed position and without involving any type of risk or bleeding accident, which substantially improves the quality of life of the user.

### Description of the invention

The first object of the present invention is to provide a retention device configured to retain the outside of a urinary catheter or feeding catheter in a fixed position on an external part of the user's body, and which overcomes the aforementioned drawbacks and presents the advantages described below.

The second object of the present invention is to provide an assembly formed by this retention device and by a urinary catheter or "T" shaped feeding catheter.

According to a first aspect, the present invention provides a retention device configured to be coupled to the end outer portion of a urinary catheter or feeding catheter and retain this end outer portion on an element external to the user's body, wherein the retention device is characterized by comprising a body having:
- a first portion extending in a longitudinal direction "X" having an inner recess configured to receive and accommodate an end portion of a urinary catheter or feeding catheter, and
- a fastening element arranged in the first portion, the fastening element being configured to releasably fasten the retention device to an element external to the user's body.

From now on, the term catheter will be used to refer to a urinary catheter or a "T" shaped feeding catheter.

Advantageously, thanks to the provision of this fastening element for releasably fastening the retention device to an element external to the user's body, it is possible to house inside the retention device an end part of the part external to the patient's body of the catheter and to fasten, in a releasable manner, the free end that is retained in the retention device to an element external to the user's body. This element external to the user's body may be, for example, an elastic band placed on the user's leg or their underwear. This allows the user, on the one hand, to protect the catheter from possible impacts or blows that it may suffer during use while, on the other hand, the free end of the catheter can be comfortably hung according to the specific needs of the user, for example, when the catheter is in its closed position. For example, the user may hang the retention device with the end portion of the catheter inserted into the retention device in the face-up position of the upper edge of the underwear, or may hang the retention device with the end portion of the catheter inserted in the opposite position, i.e. in the face-down position, on an elastic band that is disposed surrounding the leg of the user.

According to a preferred embodiment of the present invention, the retention device is configured to retain a catheter, such as a urinary tube or a feeding tube, in an element external to the user's body, and is characterized by comprising a body which in turn comprises:
- a first portion extending in a longitudinal direction "X" comprising an inner recess extending along the longitudinal direction "X", the longitudinal inner recess being configured to receive and accommodate an end portion of a catheter, wherein the first portion is closed above by an upper portion comprising a leakproof element configured to isolate the outlet port of the catheter and thereby block the fluid outlet;
- a second portion extending in a second direction "Y", transverse to the longitudinal direction "X", wherein the second portion comprises an inner recess extending along the transverse direction "Y", the transverse inner recess being configured to receive and accommodate at least a part of the sliding valve of the catheter, and wherein the second portion is open at one of its ends so as to enable a part of the sliding valve to protrude where the activation button is located, while at the opposite end presents an anti-opening wall to act as a stop for the sliding of the activation button by receiving pressure from the other end and at the same time to keep the sliding valve of the catheter in the closed position whenever it is inserted in the retention device; and
- a fastening element configured to releasably fasten the retention device to an element external to the user's body.

The retention device of the invention is preferably suitable for being coupled to the end of a "T" shaped catheter as described in European patent no. EP2872194A1, i.e. those having a urinary catheter, comprising a tube, said tube presenting at one end an introduction section for introduction through a urethra into a urinary bladder, and presenting at the opposite end a connection section for connecting connection elements, wherein through the introduction section fluid can be evacuated from the urethra into the connection section, a valve being arranged between the introduction section and the connection section that can be closed, so that fluid can be prevented from leaving the connection section when the valve is closed, wherein the valve is preferably configured as a slide valve and has three switching positions, the valve being closed in a closed position, and wherein the valve is air-permeable in a central position, and wherein the valve is open in a release position. However, this retention device can be perfectly used for other types of similar urinary catheters, such as a transurethral bladder catheter, a permanent type catheter, or a wash type catheter; and with another type of valve other than a three-position switching slide valve. This retention device may also be used in combination with feed catheters having a valve similar to that shown in European Patent No. EP2872194A1, i.e., having a "T" shaped valve with two or more switching positions.

The retention device for a catheter of the present invention has a body that can be rigid or semi-rigid (i.e., with a certain flexibility).

Preferably, the body of the retention device is monobloc (i.e., where the first and second portions and the fastening element are integrated into one and the same body), which allows the user to insert the end part of the urinary catheter under pressure and easily into the retention device and to be contained inside it without moving by being fitted tightly inside it, and at the same time allows to protect the end part of the valve against possible blows. In the same way, the user can easily remove the end part of the urinary catheter from the retention device thanks to the fact that the body of the retention device has a certain flexibility that allows the user to remove the end of the valve from its interior.

The retention device is preferably made of a polymer, such as a thermoplastic polymer, although other different materials can also be employed, as long as they provide a certain rigidity to prevent the tab from accidentally moving and the device from being actuated.

Preferably, the preferred embodiment of the present invention presents the fastening element arranged, in a fixed manner, in the first portion, and more specifically in its rear part, although it could also be arranged in the second portion.

Preferably, the fastening element of the retention device is completely integrated in the first portion, forming a monobloc body.

According to a preferred embodiment of the invention, the fastening element is configured by a tab arranged on the rear part or back of the fastening element (i.e. on the opposite side to where the longitudinal inner recess of the first portion is located) and the fixed upper end of the tab emerging from the upper part of the first portion of the retention device, which guarantees easy and comfortable connection and release of the retention device by the user to an external element, such as a strap, trousers or other clothing or device, thus avoiding accidental pulling of the catheter inserted therein. However, other conventional fastening means equivalent to a hanging tab may be possible, without altering the essence of the present invention.

According to a preferred embodiment of the invention, the first portion has an elongated body in the form of a half-barrel, which is closed above by an upper portion covering the upper part of the half-barrel, and where the longitudinal gap existing inside the groove has dimensions such as to receive, in a tight manner, an end part of the connection section of a urinary catheter. This upper portion has the function of supporting an anti-leakage element, where the function of the anti-leakage element is to isolate the outlet port of the catheter. The half-rod shape allows a longitudinal strip of the outer end portion of the catheter to protrude outward, which helps the simple and convenient insertion of the outer end portion of the catheter in this first portion. The end part of the catheter may have a cylindrical or slightly frustoconical section.

The inner shape of the first half-barrel enveloping portion may be frustoconical or have any other possible configuration, depending on the outer shape of the end portion of the catheter. That is, the first portion will have an inner space with the same shape and size as the end part of the catheter for which it was designed.

Preferably, the first portion is formed by a hollow body provided with a thin wall.

This tight configuration surrounding the outermost part of the catheter, together with the rigid or semi-rigid material of the retention device, and together with the more flexible material of the end part of the catheter, allows the user to comfortably place the outer end part of the catheter inside the retention device, exerting a slight pressure and leaving the end part of the catheter in a fixed position without being easily released. Likewise, the user can easily remove the outer end part of the retention device, when desired.

Preferably, the anti-leakage element or sealing element is configured by a protruding element on the inside of the upper portion of the first portion of the retention device, and wherein the anti-leakage element is configured to fit perfectly inside the end opening of the catheter or urinary catheter connection tube, thereby sealing it and preventing any liquid from reaching the exterior of the retention device.

Preferably, the protruding element is configured by a convex protrusion, of a substantially semi-spherical configuration. Optionally, this protrusion may be of a more flexible material than the rest of the retention device, to cushion the catheter end stop over the most protruding point of the protruding element.

According to the preferred embodiment of the invention, the urinary catheter retention device of the invention further comprises a second portion extending in a second direction "Y", transverse to the longitudinal direction "X" of the first portion, wherein the second portion has an inner recess to receive and accommodate tightly at least one part of the sliding valve of a urinary catheter.

Preferably, the second portion comprises an enveloping elongated body provided with an inner recess with dimensions to receive, in a tight manner, at least a part of the sliding valve of the urinary catheter (which runs perpendicular to the end part of the urinary catheter), and at one end of the elongated body it has an opening so that a part of the sliding valve where the activation button is located can protrude, while at the opposite end of the elongated body it has an anti-opening wall configured to cover this end of the second portion and act as a stop for the sliding of the activation button when receiving pressure from the other end, at the same time keeping the sliding valve in a closed position all the time that it is inserted in the retention device. The provision of this anti-opening wall allows it to receive pressure on the valve without accidental actuation, which is a great advantage.

Preferably, the second transverse portion has a hollow body, with a wall of a thickness such as to block the movement of the actuator button of the sliding valve when a force is applied on it such that, without the presence of the wall, the actuator button would move.

Preferably, the anti-opening wall comprises a small opening, the function of which is to be able to house a tactile system that is arranged to be used for people with reduced vision, for example, a small protrusion that allows to recognize by touch whether the device is in the open or closed position. This opening extends in a particular direction of the anti-opening wall and occupies less than half the total area of the wall. This wall may have different exterior shapes, for example, an arcuate contour and a reduced thickness similar to the thickness of the wall of the rest of the body of the second portion.

Preferably, the body of the second portion comprises an opening in the lower central part, wherein the lower opening is aligned with the first portion. This lower opening is necessary so that the longitudinal end of the urinary catheter can pass through the interior and through the second portion until it reaches the first portion. This lower opening has the shape and size necessary to receive, in a tight manner, the longitudinal end of the catheter, i.e., for example the lower opening has a circumferential arc contour, such as a half-circumference.

This second portion allows to partially wrap at least a part of the sliding valve, so that the catheter with its valve is completely inserted inside the pressure retention device, and acts as a protector against possible impacts or blows, in addition to other functions.

According to a second aspect, the present invention provides an assembly formed by a urinary catheter and a urinary catheter retention device, comprising:
- a urinary catheter, comprising a tube , the tube presenting at one end an introduction section for introduction through a urethra into a urinary bladder, and presenting at the opposite end a connection section for connecting connection elements, wherein fluid can be evacuated from the urethra through the introduction section to the connection section, a valve that can be closed being arranged between the introduction section and the connection section, such that fluid can be prevented from leaving the connection section when the valve is closed, wherein the valve is configured as a slide valve and has three switching positions, the valve being closed in a closed position, and wherein the valve is air-permeable in a central position, and wherein the valve is open in a release position; and
- a urinary catheter retention device, comprising a body with:
   - a first portion extending in a longitudinal direction "X" and having an inner recess for receiving and accommodating an end part of a urinary catheter, and
   - a fastening element arranged in the first portion and configured to releasably fasten the retention device to an element external to the user's body.

According to a preferred embodiment the assembly formed by a urinary catheter and a retention device for the urinary catheter, is characterized by comprising:
- a urinary catheter comprising a tube, the tube presenting at one end an introduction section for introduction through a urethra into a urinary bladder of the patient, and presenting at the opposite end a connection section for connecting connection elements, wherein fluid can be evacuated through the introduction section from the urethra into the connection section, a valve that can be closed being arranged between the introduction section and the connection section, such that fluid can be prevented from leaving the connection section when the valve is closed, wherein the valve is configured as a slide valve and has three switching positions, the valve being closed in a closed position, and wherein the valve is air-permeable in a central position, and wherein the valve is open in a release position; and
- a urinary catheter retention device, comprising a body that comprises:
   - a first portion extending in a longitudinal direction "X" and comprising an inner recess extending along the longitudinal direction "X" configured to receive and accommodate an end part of a catheter, wherein the first portion is closed above by an upper portion comprising a leakproof element configured to isolate the outlet port of the catheter and thereby block the fluid outlet;
   - a second portion extending in a second direction "Y", transverse to the longitudinal direction "X", wherein the second portion comprises an inner recess extending along the transverse direction "Y" to receive and accommodate at least a part of the catheter sliding valve, and wherein the second portion is open at one of its ends so that a part of the catheter sliding valve where the activation button is located can protrude, while at the opposite end of the elongated body it presents an anti-opening wall to act as a stop for the sliding of the activation button by receiving pressure from the other end and at the same time to keep the catheter sliding valve in the closed position all the time it is fitted inside the retention device; and
   - a fastening element configured to releasably fasten the retention device to an element external to the user's body.

Preferably, in the urinary catheter plus retention device assembly the fastening element is arranged in the first portion.

An air-permeable filter and/or membrane section is provided in the sliding valve of the urinary catheter.

Preferably, the urinary catheter at its far end of the connection section has a closed hollow tip with at least two openings facing each other according to the Nelaton style.

Alternatively, the urinary catheter is a transurethral catheter for the bladder.

Alternatively, the urinary catheter is a permanent type catheter.

Alternatively, the urinary catheter is a wash-type catheter.

Advantageously, the urinary catheter may be made of rubber and/or latex and/or polyvinyl chloride PVC and/or silicone or other materials having similar properties.

### Brief description of the drawings

For a better understanding of the description made herein, a set of drawings has been provided wherein, schematically and solely by way of a non-limiting example, a practical case of an embodiment of the retention device of the invention is represented.
Figures 1 to 9 show different views of an example of embodiment of the retention device, wherein:
   Figure 1 is a front elevation view,
   Figure 2 is a rear elevation view,
   Figure 3 is a left side elevation view,
   Figure 4 is a right side elevation view,
   Figure 5 is a top plan view,
   Figure 6 is a bottom plan view,
   Figure 7 is a rear perspective view,
   Figure 8 is a front left perspective view, and
   Figure 9 is a right front perspective view.
Figures 10 to 16 show different views of the example of embodiment of the retention device of figures 1 to 9 in which the free end part of the urinary catheter has been inserted, wherein:

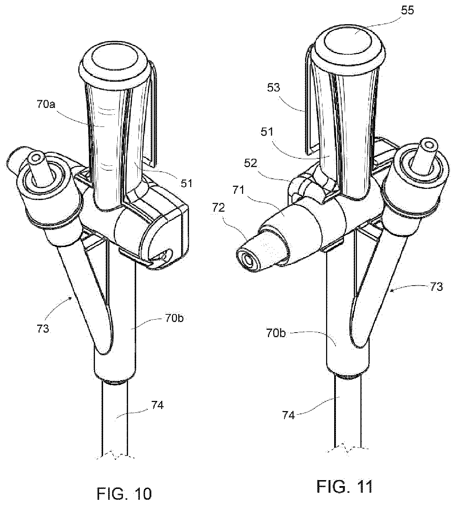

   Figure 10 is a right front perspective view,
   Figure 11 is a front left perspective view,

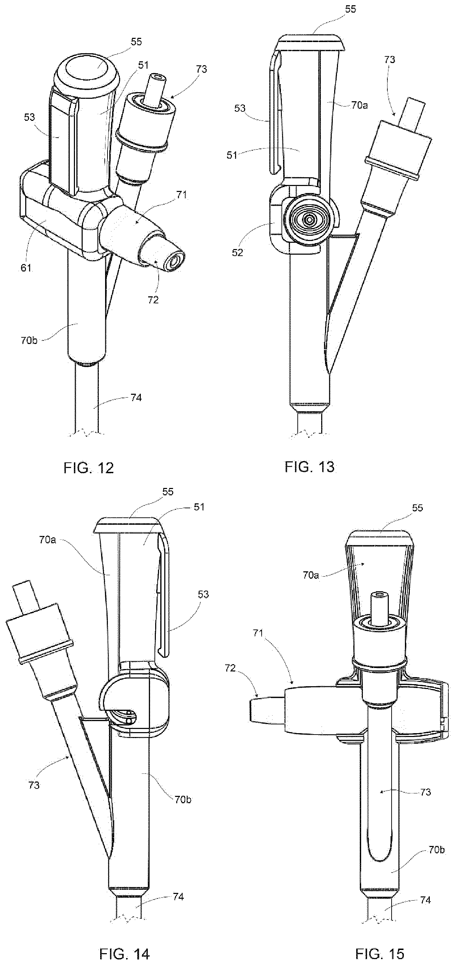

   Figure 12 is a rear perspective view,
   Figure 13 is a left side elevation view,
   Figure 14 is a right side elevation view,
   Figure 15 is a front elevation view, and

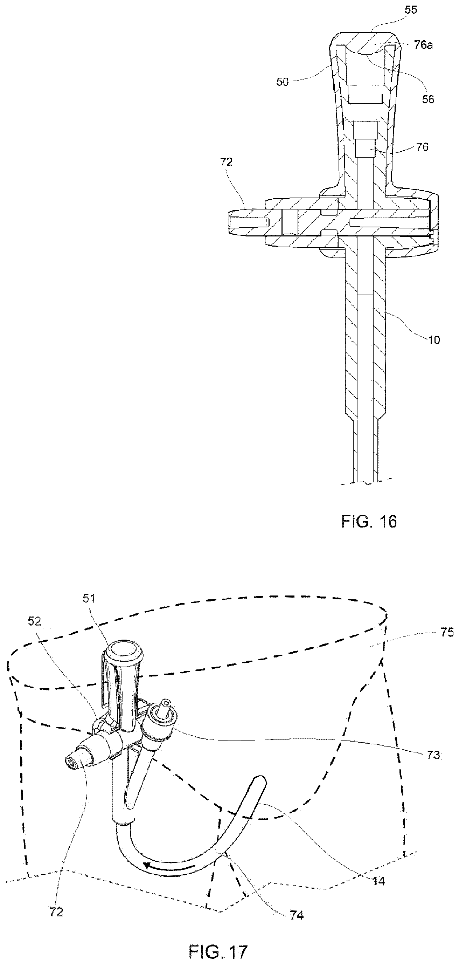

   Figure 16 shows the same view as the previous figure, in a longitudinal section.

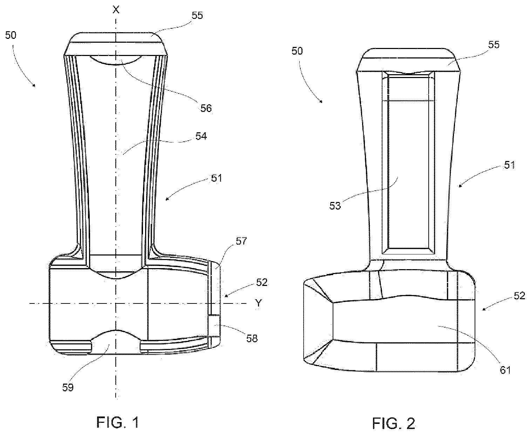

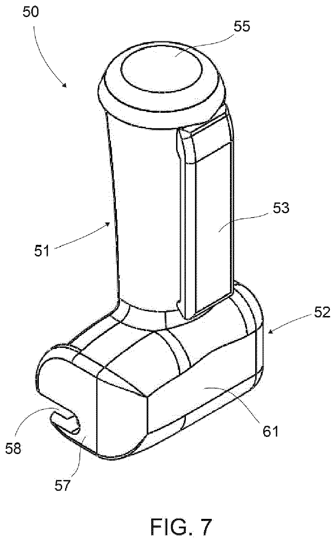

Figure 17 shows a left front perspective view of the embodiment of the retention device of figures 1 to 9 with the free end part of the urinary catheter inserted into and in use position on a user, i.e., wherein the tab is hanging, for example, from the top of the user's underwear.

### Description of some examples of embodiment

Some examples of embodiments of the present invention are described below with reference to figures 1 to 17.

As shown in figures 1 to 9, according to a possible example of the present invention, a retention device (50) is provided configured to be coupled to the free end of a urinary catheter and retain this free end on an element external to the user's body, wherein the retention device (50) is formed by a rigid body having a monobloc body with:
- a first portion (51) extending in a longitudinal direction "X" and having an inner recess (54) for receiving and accommodating an end part of a urinary catheter,
- wherein from the rear part and upper end of the first portion (51) emerges a tab (53) configured to releasably fasten the retention device (50) to an element external to the user's body, and
- a second portion (52), which extends in a second transverse "Y" direction, and wherein the second portion (52) has an interior recess (62) for receiving and accommodating at least a portion of the sliding valve of the urinary catheter.

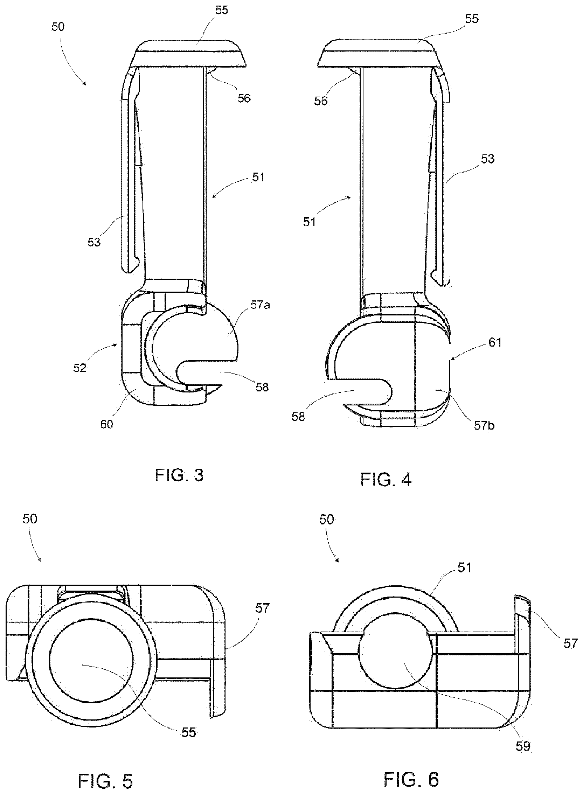

In this embodiment, the tab (53) extends from the uppermost end of the first portion (51) downward to almost the lowermost portion of the first portion 51. The extension direction of the tab (53) is slightly inclined with respect to the longitudinal direction "X", that is, the tab (53) moves slightly away from the surface of the first portion (51) as it extends downwards (see figures 3 and 4).

In this embodiment, the first portion (51) is constituted by an elongated half-barrel body (i.e. with a "C" shaped cross section), which is closed at the top by a stop portion (55) and where the recess (54) inside the groove has dimensions such as to receive, in a tight manner, a part of the connection section (70a) of a urinary catheter. This stop portion (55) at the top has the function of supporting an anti-leakage element to isolate the outlet port of the catheter. As shown in Figure 1, the first portion (51) decreases in width from the upper part to the lower part where it is coupled to the second portion (52), that is, it has a body in the form of a conical throne segment.

The stop portion (55) is formed by a flat element of low thickness that acts as a support for an anti-leakage element (56), with a convex surface, which protrudes from the inner part of the stop portion (55) of the first portion of the retention device, see Figures 1, 3 and 4. This stop portion 55 may have, for example, a circular shape (see Figure 5).

Preferably, the anti-leakage element (56) or sealing element is configured by a protruding element on the inner part of the stopper portion (55) of the first portion (51) of the retention device, and wherein the anti-leakage element (56) is configured to fit perfectly inside the extreme opening of the catheter or urinary catheter connection tube, thereby producing its sealing and thereby preventing any amount of liquid from leaving the outside of the retention device. Figure 16 shows how the anti-leakage element (56), with a slightly semi-circular contour, has entered through the opening and is mounted inserted in the end of the catheter or urinary catheter connection tube.

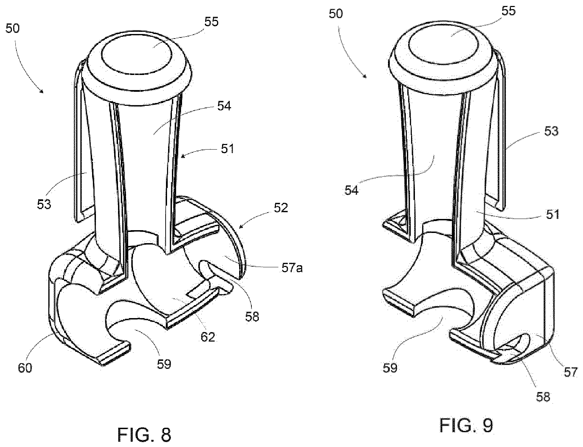

In this example of embodiment, the second portion (52) is constituted by an elongated body extending in a transverse direction, but of shorter length than the first portion (51), also shaped as a half-barrel (i.e., with a "C" -shaped cross section, see figure 3). The rear part of the half-rod-shaped surface is represented by (61) and the inner recess by (62), where the inner recess (62) that is created inside this "C" shaped section has dimensions such as to receive, in a tight manner and inserted under pressure, at least a part of the sliding valve (71) of the urinary catheter (which corresponds to the part where the activation button (72) is located). Further, as clearly seen in Figures 8 to 9, the transverse body of the second portion (52) is open at one of its ends, forming an open left side section (60) so that a portion of the slide valve (71) can protrude along with the activation button (72) that is at the end. On the other end, the transverse body of the second portion (52) is closed by an anti-opening wall (57), wherein the anti-opening wall (57) has an inner face (57a) and an outer face (57b), and has a small opening (58) at its bottom, wherein this opening (58) is for accommodating a tactile recognition system of the position of the catheter for use by people with reduced vision. In addition, the second portion (52) has an opening (59) in the form of a "C" -shaped inlet in its central and lower part for the passage of the lower connection section (70b) of the catheter. Figure 1 clearly shows that the central opening (59) is fully aligned with the longitudinal axis "X" of the first portion (51).

With reference to figures 10 to 16, the same example of a retention device (50) as the previous figures are depicted therein, but in this case with the free end part of a urinary catheter (10) conveniently inserted into the retention device (50). In the specific case illustrated, a urinary catheter (10) known as a two-way catheter is shown, where only the free end of the catheter and a small part of the flexible tube (74) have been depicted, but not the rest of the flexible tube (74) and the introduction section (14), shown in figure 17, through a urethra in a user's urinary bladder. The urinary catheter (10) has at its free end or connection end a valve (71-72) configured as a slide valve in the form of a plunger that moves inside a cylinder, arranged transversely. In the case of the urinary catheter (10) shown, this is a two-way type catheter. Figures 10 to 16 of the catheter (10) also show a junction section (73) that connects a balloon arranged at the introduction end (not shown) that inflates with a liquid that enters through a secondary channel of the tube towards the introduction end where the balloon is. At the end of the body 70a an opening is formed in which a connection element (not depicted in the figures), such as a urine bag, can be connected. Using this balloon, the urinary catheter (10) can be fixed to the urinary bladder in its position.

This specification does not explain the internal functioning of the urinary catheter (10) or the valve (71-72), since it is the same as that described in European patent no. EP2872194A1.

In particular figure 16 shows a possible configuration of the end part of the catheter (10), which has an end provided with a central orifice (76) in a staggered form that decreases in diameter, and which ends above in a mouth (76a). In this figure it is clearly seen how the convex protuberance (56) fits perfectly within the upper mouth (76a) of the central orifice (76).

Figure 16 also shows how the part opposite the button of the valve (71-72) is arranged in contact with the inner face (57a) of the anti-opening wall (57). In this position the valve is closed, that is, preventing the passage of fluid through the interior of the central orifice (76).

In particular figure 17 shows the retention device (50) with the end part of the urinary catheter inserted therein, which is hung upside down by means of the tab on the top of the underwear (75) of a user. It is seen how the tube (74) is hidden underneath the underwear until reaching the bladder. The direction of urine flow towards the discharge end has also been illustrated. In this way, by being fixed at a point of the user's clothing (75) such that the tube (74) is not uncomfortable, it is possible to prevent the undesirable traction of the tube (74), which generally causes injury and bleeding of the user, allowing the user to perform a physical activity with total normality and comfort.

Although reference has been made to a specific embodiment of the invention, it is clear to a person skilled in the art that the retention device and retention device assembly described are susceptible to numerous variations and modifications, and that all the details mentioned can be replaced by other technically equivalent ones, without departing from the scope of protection defined by the attached claims.

## Claims

1. Retention device (50) configured to retain a catheter, such as a urinary catheter or a feeding catheter, in an element external to the user's body, for example, in a strap or user's underwear, **characterized in that it** comprises a body with:
- a first portion (51) extending in a longitudinal direction "X" and comprising an inner recess (54) extending along the longitudinal direction "X", wherein the first portion (51) is closed above by an upper portion (55) comprising an anti-leakage element (56) of the open end of the catheter (10) or catheter connection tube;
- a second portion (52) extending in a second direction "Y", transverse to the longitudinal direction "X", wherein the second portion (52) comprises an inner recess (62) extending along the transverse direction "Y", and wherein the second portion (52) is open at one of its ends, while at the opposite end comprising an anti-opening wall (57); and
- a fastening element (53) releasably fastening the retention device (50) to an element external to the user's body.

2. Retention device (50) for a catheter, according to claim 1, wherein the fastening element (53) is arranged in the first portion (51).

3. Retention device (50) for a catheter, according to claim 1 or 2, wherein the fastening element (53) is integrated in the first portion (51).

4. Retention device (50) for a catheter, according to claim 1, 2 or 3, wherein the fastening element (53) is a tab, the upper end of the tab emerging from the rear face of the retention device (50).

5. Retention device (50) for a catheter, according to claim 1, wherein the first portion (51) has an elongated body in the form of a half-barrel.

6. Retention device (50) for a catheter, according to claim 1, wherein the anti-leakage element (56) is configured by a protruding element on the inner part of the upper portion (55) of the first portion (51), and wherein the anti-leakage element (56) is configured to fit perfectly inside the open end of the catheter or the catheter connection tube.

7. Retention device (50) for a catheter, according to claim 1 or 6, wherein the anti-leakage element (56) is a semi-spherical protrusion.

8. Retention device (50) for a catheter, according to claim 1, wherein the second portion (52) has an elongated half-barrel-shaped body, the inner recess (62) being created to receive inside and accommodate, in a tight manner, at least a part of the sliding valve (71-72) of a catheter.

9. Retention device (50) for a catheter, according to claim 1, wherein the anti-opening wall (57) comprises an opening (58).

10. Retention device (50) for a catheter, according to claim 1, wherein the body of the second transverse portion (52) comprises an opening (59) in the form of a "C" in the central and lower part, wherein the opening (59) is aligned with the longitudinal axis "X" of the first portion (51).

11. Retention device (50) for a catheter, according to claim 1, wherein it has a monobloc body.

12. Assembly consisting of a urinary catheter (10) and a retention device (50) for the urinary catheter (10), **characterized in that it** comprises:
- a urinary catheter (10) comprising a tube (74), the tube (74) having at one end an introduction section for introducing it through a urethra into a urinary bladder of the patient, and having at the opposite end a connection section (70a-70b) for connecting connection elements, wherein through the introduction section fluid can be evacuated from the urethra into the connection section (70a-70b), a valve that can be closed (71-72) being arranged between the introduction section and the connection section (70a-70b), such that fluid can be prevented from leaving from the connection section (70a-70b) when the valve is closed (71-72), wherein the valve (71-72) is configured as a slide valve and has three switching positions, the valve (71-72) being closed in a closed position, and wherein the valve (71-72) is air-permeable in a central position, and wherein the valve (71-72) is open in a release position; and
- a retention device (50) for the urinary catheter (10), comprising a body with:
- a first portion (51) extending in a longitudinal direction "X" and having an inner recess (54) extending along the longitudinal direction "X", wherein the first portion (51) is closed at the top by an upper portion (55) comprising an anti-leakage element (56) of the open end of the catheter (10) or catheter connection tube;
- a second portion (52) extending in a second direction "Y", transverse to the longitudinal direction "X", wherein the second portion (52) has an inner orifice (62) extending along the transverse direction "Y", and wherein the second portion (52) is open at one of its ends, while at the opposite end it comprises an anti-opening wall (57); and
- a fastening element (53) that releasably fastens the retention device (50) to an element external to the user's body.

13. Urinary catheter assembly (10) and retention device (50), according to claim 12, wherein the fastening element (53) is arranged in the first portion (51).
